# EUROPEAN PATENT SPECIFICATION

(11) **EP 0 649 217 B1**
(45) Date of publication and mention of the grant of the patent: **08.09.1999**
(21) Application number: 94307397.3
(22) Date of filing: 10.10.1994
(51) Int. Cl.: H02P 6/08, A61B 17/32, H02H 7/093

(54) **Brushless motor**
Bürstenloser Motor
Moteur sans balai

(30) Priority: 12.10.1993 US 135297
(43) Date of publication of application: 19.04.1995
(73) Proprietor: SMITH & NEPHEW, INC., Memphis, Tennessee 38116 (US)
(72) Inventor: Krause, Kenneth W., Sandown, New Hampshire 03873 (US)
(74) Representative: Regan, Heather

(56) References cited:
- EP-A- 0 148 269
- EP-A- 0 566 297
- DE-A- 2 757 132
- GB-A- 2 156 172
- US-A- 4 867 155
- US-A- 5 017 846
- PATENT ABSTRACTS OF JAPAN vol. 10, no. 298 (P-505) 9 October 1986 & JP-A-61 114 304 (FUJITSU LTD) 2 December 1986
- ELECTRONIC ENGINEERING, vol.58, no.719, November 1986, LONDON GB pages 51 - 59 PHIL DAVIES ET AL. 'Three phase control and drive IC for brushless motors'
- MACHINE DESIGN, vol.60, no.13, 9 June 1988, CLEVELAND US pages 140 - 144, XP000100657 ROBERT BENZER 'Single-chip brushless-motor controller'
- INDUSTRY APPLICATIONS SOCIETY . IEEE-IAS-195 ANNUAL MEETING., November 1985, TORONTO, CANADA pages 536 - 541 D.A. TOPMILLER ET AL. 'A microprocessor-based controller for oscillatory mode drive of a tubular brushless DC motor'
- PATENT ABSTRACTS OF JAPAN vol. 13, no. 295 (E-783) 7 July 1989 & JP-A-01 074 089 (HITACHI LTD) 20 March 1989

## Description

### BACKGROUND OF THE INVENTION

### 1. Field of the Invention

This invention generally relates to an all-digital motor control system and, more particularly, to a system for controlling the speed or armature position of a motor.

### 2. Description of Related Art

Speed control systems for controlling the speed of motors are generally known. However, such systems rely, at least in part, on analog signals and an analog-to-digital converter to convert the analog signals to digital signals for subsequent processing by digital signal processors. This adds hardware complexity and rigidity to the overall system. Also, the reliance on analog signals, at least in part, introduces an element of inaccuracy in motor speed control.

It is a general object of this invention to advance the state of the art of control for motors.

It is another object of this invention to reduce the hardware requirement and system rigidity in such control systems.

Another object of this invention is to provide all digital motor control systems, and with the attendant advantage of accuracy and speed of response.

Another subject of the invention is to provide a motor control system which has particular application in surgical procedures.

Another is to provide a system which accurately controls surgical pumps, and motor driven surgical tools.

In our earlier European patent application EP-A-566297, we disclose an all-digital brushless motor control system wherein the controller includes a single look-up table having bit patterns wherein each pattern corresponds to a different armature position.

In accordance with the present invention, there is provided an all-digital control system for a motor having an armature, comprising:
(a) a main digital signal processor for supplying a digital input command signal indicative of a desired motor operation;
(b) a drive controller in digital communication with the main processor, for generating, for each phase of the motor, and in response to the command signal, a digital commutation signal to move the armature, and a digital pulse width modulated signal having a duty cycle established by the input command signal;
(c) switching means in digital communication with the controller, for generating, for each phase, and in response to each commutation signal and each pulse width modulated signal, a digital two-state control signal having an on-state which lasts for said duty cycle;
(d) means in digital communication with the controller, for generating, for each phase, a digital tachometer signal indicative of armature position;
(e) said controller being further operative for processing the tachometer signal, to generate a digital output signal indicative of the actual armature speed or position, and for communicating the digital output signal to the main processor, wherein controller includes a first look-up table having bit patterns corresponding to different armature positions and second look-up table for modulating said pulse width modulated signal.

In keeping with these objects, and others which will become apparent hereinafter, one feature of this invention resides, briefly stated, in an all-digital control system for a motor having an armature. The system comprises a main digital signal processor for supplying a digital command signal indicative of a desired motor operation. A drive controller in direct digital communication with the main processor generates, for each phase, and in response to the command signal, a digital commutation signal to move the armature with a digital pulse width modulated signal having a duty cycle established by the input command signal.

The system further comprises switching means, e.g. a multi-phase bridge, in digital communication with the controller. The bridge is operative for generating, for each phase, and in response to each commutation signal and each pulse width modulated signal, a digital two-state control signal having an on-state which lasts for the duty cycle.

The system still further comprises means in digital communication with the controller, for generating, for each phase a digital tachometer signal indicative of armature position. The controller is further operative for processing the tachometer signal to generate a digital output signal indicative of the actual armature speed or position. The controller directly digitally communicates the output signal to the main processor.

In a preferred embodiment, the main processor and the drive controller are interconnected by, and digitally communicate through, a plurality of optical fibers. No analog signals and, or course, no analog-to-digital converters, are used anywhere in the speed control systems, thereby simplifying the hardware requirement for such system, and also eliminating any inaccuracies due to the presence of analog signals.

Another feature of this invention resides in shutting down the system upon the elapse of a predetermined time during which no input signal is received by the controller.

The control system has particular applications for surgical equipment, e.g. for accurately controlling pumps used to maintain pressure of saline solution inside a body cavity during an operation, and for motor driven surgical drills, saws, rasps, scalpels, scissors; and for limiting torque on the motor driven surgical instruments to avoid breakage and shattering of the instruments especially when inside a patient.

The novel features which are considered as characteristic of the invention are set forth in particular in the appended claims. The invention itself, however, both as to its construction and its method of operation, together with additional objects and advantages thereof, will be best understood from the following description of specific embodiments when read in connection with the accompanying drawings.

### BRIEF DESCRIPTION OF THE DRAWINGS

FIG. 1 is a general block diagram of the overall all-digital speed control system according to this invention;
FIG. 2 is a detailed electrical schematic of the system of FIG. 1;
FIG. 3 is a flow chart depicting part of the operation of the controller;
FIG. 4 is a flow chart depicting another aspect of the operation of the controller; and
FIG. 5 is a flow chart depicting still another aspect of the operation of the controller.
FIG. 6 is a schematic block diagram of a surgical procedure, using the system of the invention.
FIG. 7 is a perspective view of a surgical tool.
FIG. 8 is a cross-sectional view through FIG. 7.
FIG. 9 is a schematic side view of a rasp.

### DETAILED DESCRIPTION OF THE PREFERRED EMBODIMENT

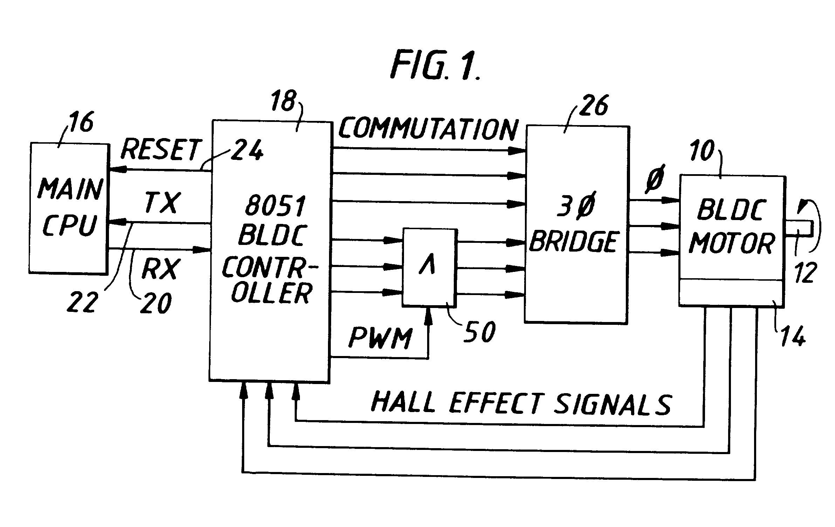

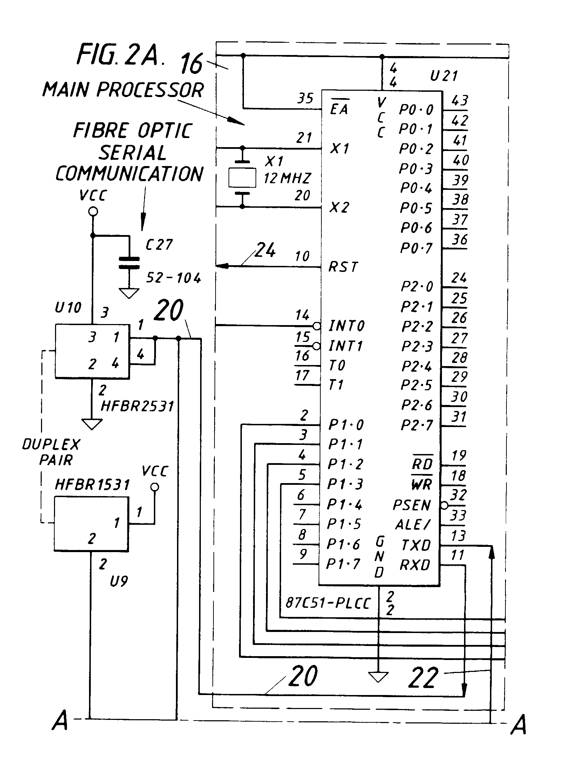

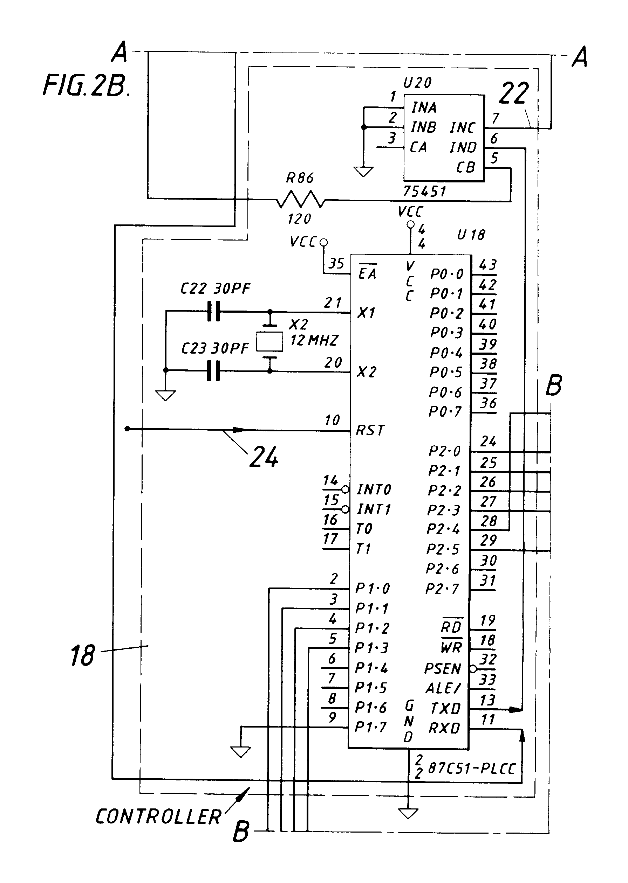

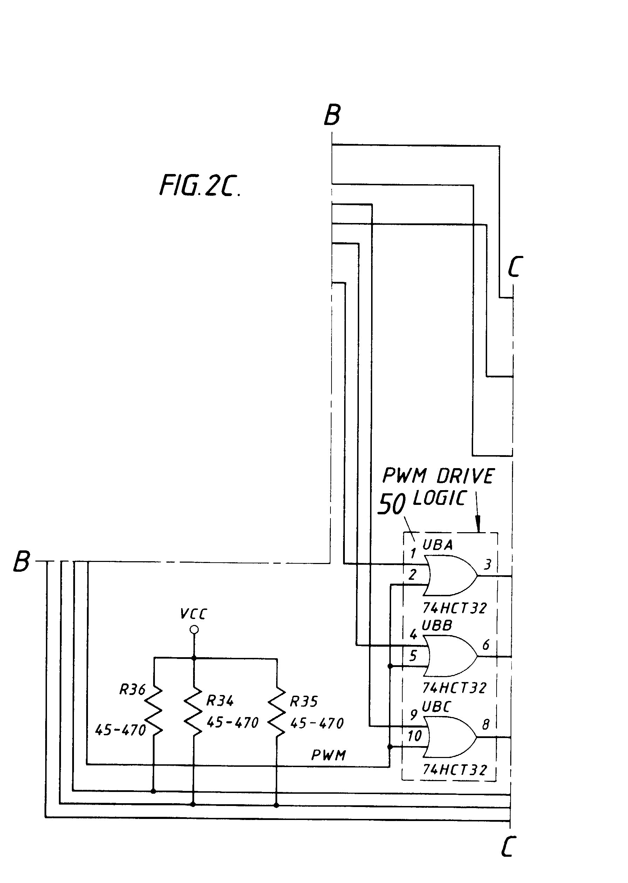

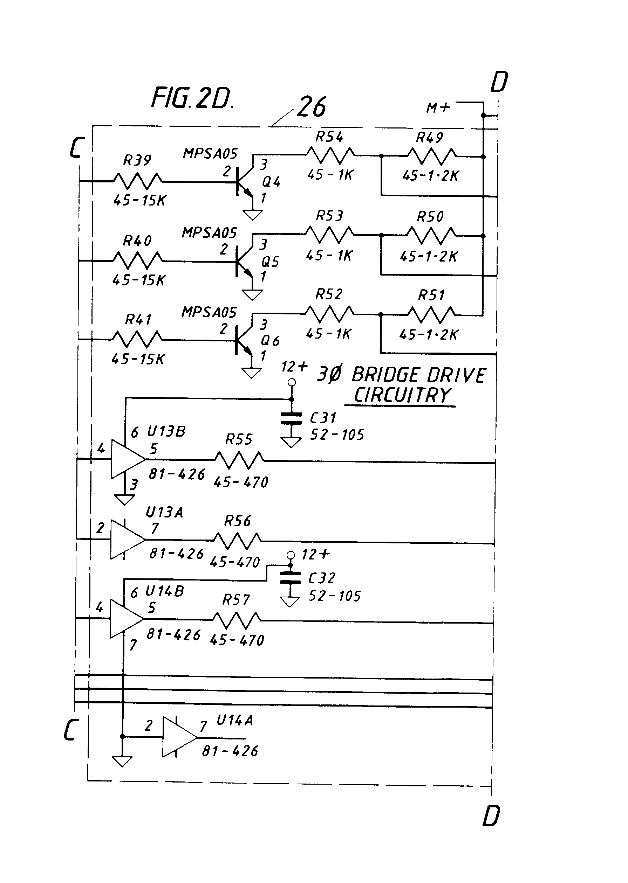

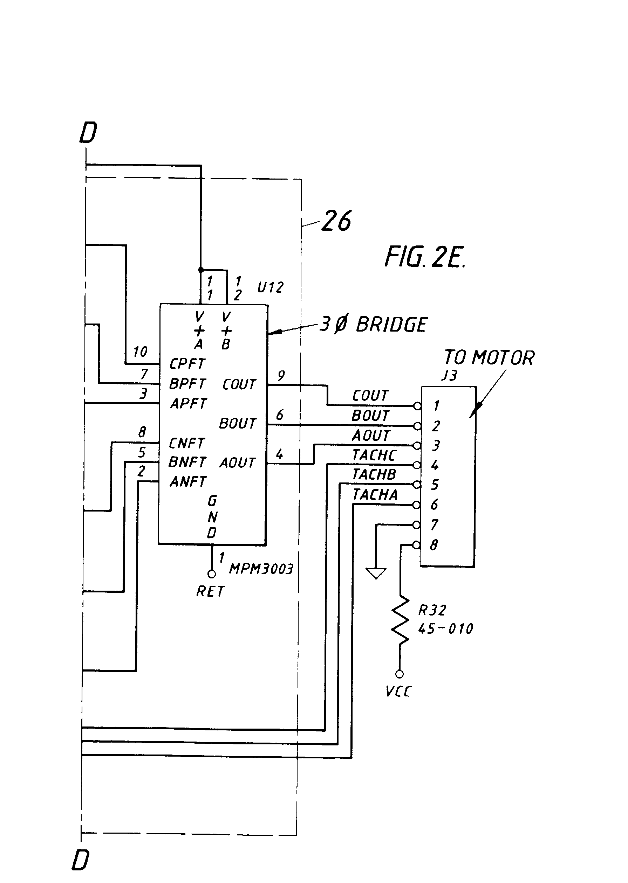

The present invention is illustrated in terms of a control system for controlling the speed of a brushless three-phase, DC motor. Referring now to the drawings, FIG. 1 is a general block diagram, and FIG. 2 is a more detailed electrical schematic, of the overall all-digital motor speed control system of this invention. Reference numeral 10 identifies a brushless, three-phase, DC motor having an armature 12. Preferably, the motor is obtained from BEI Kimco Magnetics Division of San Marcos, California, as its part No. DIH 23-20-BBNB. This motor has a plurality of conventional Hall-effect sensors 14 mounted about the armature to sense armature position.

The system includes a main digital signal processor (CPU) 16, preferably constituted as integrated circuit chip No. 87C51-PLCC. Main processor 16 is in direct digital communication with a drive controller 18, preferably also constituted as integrated circuit chip 87C51-PLCC. Processor 16 supplies a digital input speed signal RX indicative of a desired armature speed to the controller 18 over line 20. The controller 18, as will be described in detail below, supplies a digital output speed signal TX indicative of the actual armature speed to the processor 16 over line 22. Controller 18 also communicates with the processor 16 over a RESET line 24. Lines 20, 22, 24 are high speed buses capable of transmitting data at 375 kbaud. Preferably, communication lines 20, 22 and 24 are optical fibers. However, the main processor and the controller may communicate means such as a parallel communication bus, a high speed serial hardwired interface or the like.

Upon receipt of the input speed signal RX, controller 18 executes a software program as set forth on pages A-1 through A-3 of the attached Appendix. Controller 18 generates a set of six commutation signals, two for each phase of the motor, together operative for rotating the armature. More specifically, the controller includes an interior look-up table having a listing of six commutation bit patterns, each pattern representing a discrete command for the armature at an angular position spaced 60 electrical degrees from the previous armature position. The commutation signals are fed through, and processed in, a three-phase bridge circuit 26, and optionally, through a bridge driver circuit (see FIG. 2), wherein three position control signals, one for each phase, ar output to the motor 10. The Hall-effect sensors 14 sense rotation of the armature and generate two-state Hall-effect signals which advise the controller 18 when to generate the commutation signals.

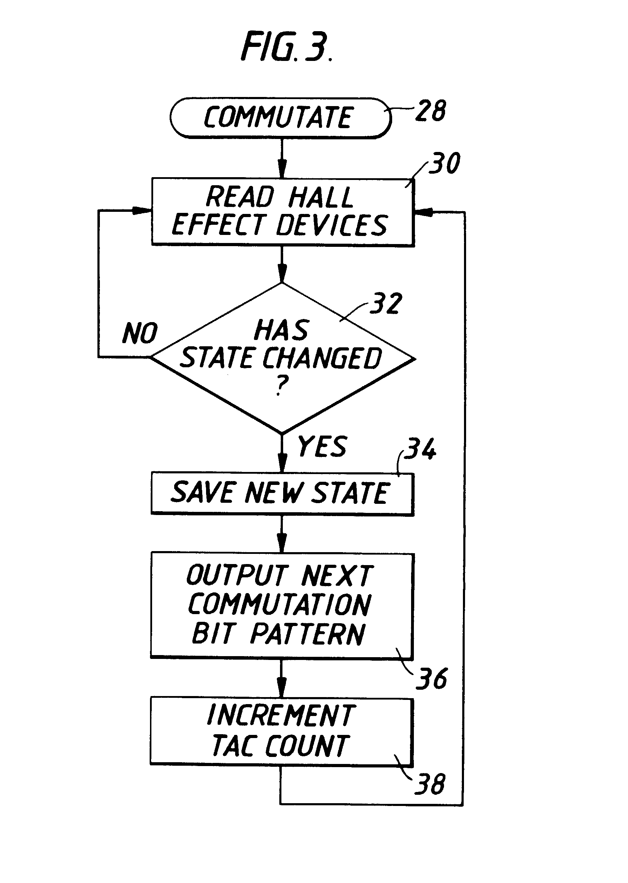

This latter aspect of the controller 18 is displayed in the flow chart of FIG. 3. The generation of the commutation signals is indicated by block 28. The reading of the Hall-effect sensors is denoted by block 30. If the controller 18 recognizes that the state of the Hall-effect signals has changed (block 32), then the new state is saved (block 34) and the next commutation bit pattern is output to the motor (block 36). Thereafter, an internal counter operative for generating a tachometer (TAC) signal is incremented (block 38) prior to the next reading of the Hall-effect sensors. The tachometer signal is eventually processed to generate the aforementioned output speed signal TX. If the state of the Hall-effect sensors did not change in block 32, this indicates that the armature has not moved 60 electrical degrees and, hence the controller attempts to read the Hall-effect sensors again in block 30.

Controller 18 also generates in response to command data from the processor 16, a digital pulse width modulated (PWM) signal having a duty cycle established by said command data. The PWM signal is carried on a carrier signal having a frequency which, in the preferred case, is 3.90625 kHz. Controller 18 has an internal software PWM timer which, in the preferred case, establishes a PWM cycle of 256 microseconds. The PWM cycle has a high and a low state. The PWM output is allowed to continue running during the high state, but is re-set to OFF in the low state. The command data controls how long the PWM timer runs; in the preferred case, from 14 - 242 µs. In this way, the duty cycle of the PWM signal is controlled from 5.47% - 94.53%.

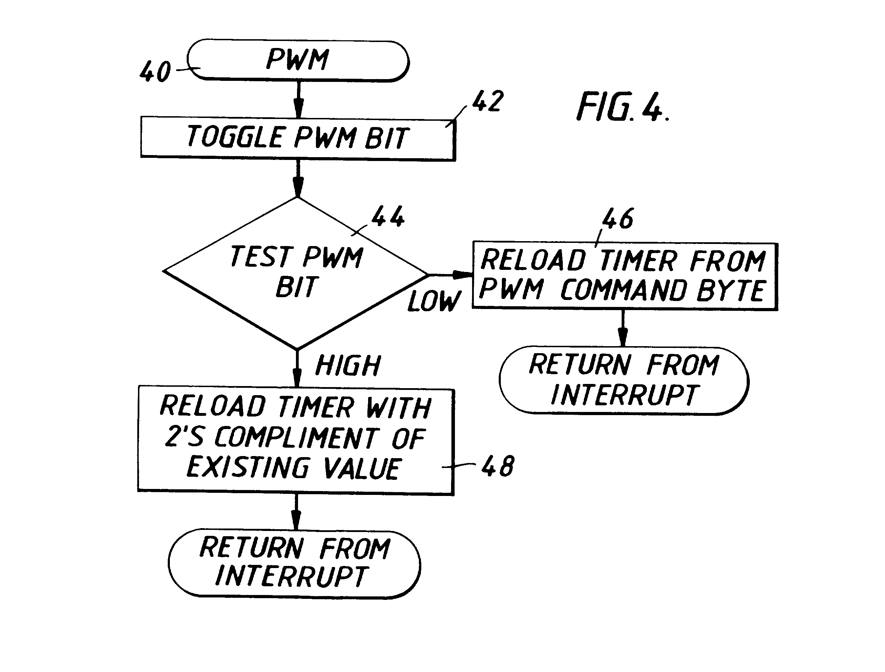

This aspect of the controller operation is depicted in FIG. 4. Block 40 represents the generation of the PWM signal. The controller toggles and generates a two-state PWM bit (block 42) and tests the state of the PWM bit in block 44. If the PWM bit has a low state, then, as depicted in block 46, the PWM timer is re-loaded from a command byte supplied by type processor 16. If the PWM bit has a high state, then the PWM timer is re-loaded with the 2's complement of its existing value (block 48).

As best shown in FIG. 1, the PWM signal is fed to a drive logic unit 50 which, as shown in FIG. 2, comprise three AND gates to which three of the commutation signals are conveyed. Unit 50 generates switching signals for the bridge 26. In turn, the bridge 26 generates, for each phase, the aforementioned modulated control signal having an on-state and an off-state.

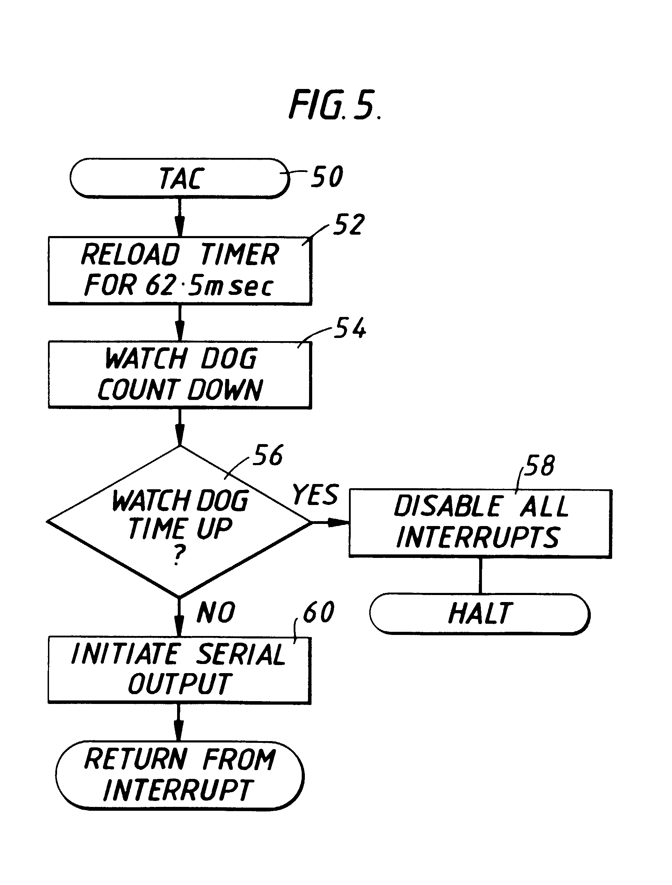

As shown in the flow chart of FIG. 5, the Hall-effect sensors, as previously mentioned, send TAC signals back to the controller (block 50) and, more specifically, TAC signals are accumulated as they occur every 62.5 ms in a TAC timer (block 52). The resulting count from the TAC counter is processed into a tachometer signal which is processed by the controller and fed back to the processor 16 over line 22, and is indicative of the actual speed of the motor.

A watchdog counter (block 54 in FIG. 5) has a pre-set count of, for example, 500 ms. Upon receipt of the TAC timer interrupt, the watchdog counter counts down. If, as determined in block 56, the 500 ms has elapsed, then the entire system is shut down (block 58). If, however, the watchdog time has not elapsed, then the command data from the processor 16 is sent to the controller over line 20 as denoted in block 60.

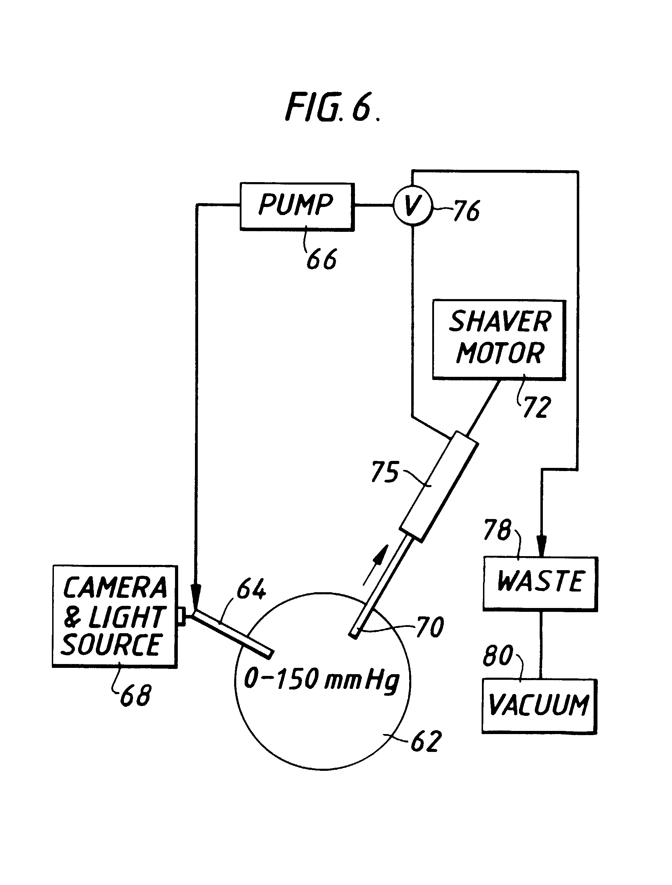

FIG. 6 is a schematic block diagram showing a setup of a typical modern surgical procedure, e.g. of an arthroscopy or laparoscopy. A joint or another area of the patient being operated on is shown at 62. A first curet 64 is introduced into the area and is attached to a source of saline solution, i.e. a pump 66 which maintains a positive pressure in the joint, e.g. at 0 to 150 mm Hg gauge. A video camera and light source 68 are also connected to the curet 64 for viewing the area and display on a T.V. monitor (not shown). A second canula 70 with a surgical instrument at its end is also introduced into the area 62. The instrument here is a shaver with a motor drive 74. The saline, blood and debris from the cutting are removed from the area through a hollow in the canula 70 and then through hose 74 which passes between a pinch valve 76 located on the pump housing 66 and which may help regulate flow from the area, and then to a waste collector 78 and to a vacuum 80 which typically maintain a pressure of 150 to 760 mm Hg absolute. Between the canula 70 and hose 74 is a tool 76 which supports the camera, the instrument therein and controls for the flow and application of vacuum.

It is important in such procedures that the pressure in the area 62 is constant. This is particularly difficult to maintain in the area of a joint where the mechanical dimensions of the joint are constantly changing, leaking and is an unstable, unsealed area. As the surgeon operates the surgical tool, opening and closing the connection to the vacuum and removing bits of tissue with bits of fluid flows, there is a constant variable, and quickly variable vacuum. It is essential for good surgical procedures that the pressure in the surgical area be constant. Particularly important is that the pressure never become too large, as this would injure the patient. Constant pressure is directly related to accurate control over the velocity of the saline flowing into the area 62. Small changes of pump speed yield very large changes in pressure. It has been found that with the control system of the present invention, a constant pressure to very tight tolerances can be made. This is particularly achieved with a pulse driven motor in the pump, whose duty cycle can be varied, and whose frequency of revolution can also be varied from a fraction of an RPM to, for example, 5000 RPM. Typical flow rates into a surgical area are from 0.0 to 2.5 liters per minute.

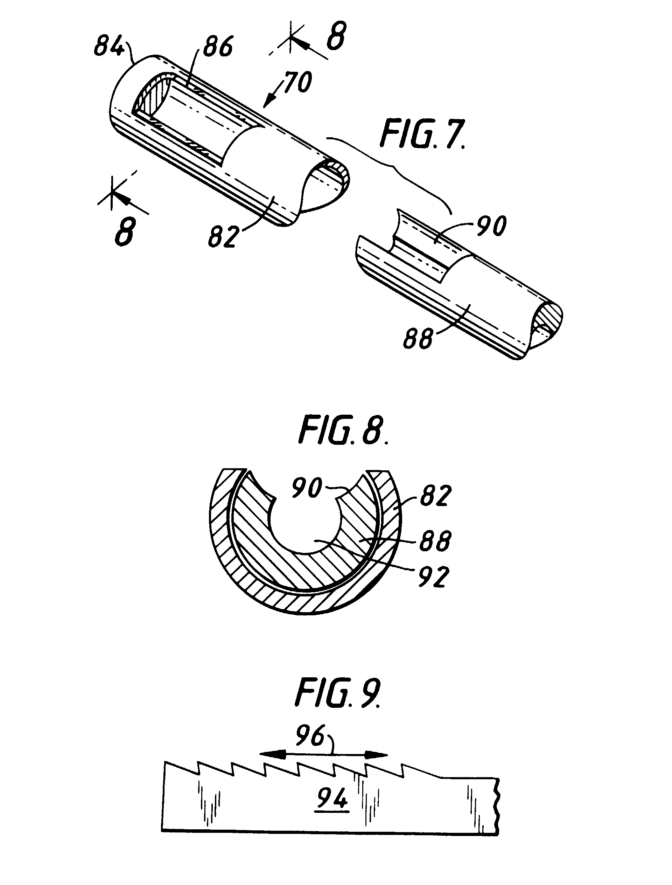

FIG. 7 is a schematic perspective, partially cut away, exploded view of part of a surgical router, which would appear at the end of the canula 70. A tube 82 closed at its distal end 84 has an opening which describes typically a cut-out section 86. The router 88 also a hollow tube, has a cutting surface with sharp edges at its distal end region 90. The router is motor driven and rotates inside the tube 82. The vacuum is drawing and fluids and debris are removed through the central hollow.

The router is typically driven at a constant speed, and rotates in one direction, driven by a motor within the shaver 72. It is desirable to control accurately the torque applied to the router, because if the torque is too large, e.g. due for example to a piece of bone or metal or other fragment getting caught in the spinning tube 88, the router itself or the tube 82, or the canula 70 may shatter with the result of spraying debris into the patient's joint. The debris, then, must be removed which is not an easy task. Also, there is an attendant trauma in the region. The control system of the present invention provides such a torque control. The system of the present invention applies a voltage or electrical drive energy, e.g., typically a series of pulses with a particular duty cycle. A digital tachometer measures the actual speed of the motor, and there is a table look-up which compares the speed with the output of the wave form for driving the router. When something gets stuck inside the curet or router the motor will normally need more power, and will thus will call for increased duty cycle in the form of more voltage or more current. The table look-up compares the duty cycle, or current, or voltage with the speed of the motor, and if the speed (it being noted that the motor and the curet are linked together), if the speed is too slow for the applied power, then the controller will drop the duty cycle, or will drop the voltage or current, and this will cut down on the torque, and thus will avoid possible fracture of the router or the tube 82. The surgeon may then observe through the camera 68 what is the condition at the end of the canula, e.g. if something is stuck, and increase the flow of saline or manipulate the tool to remove the clogging; and if need be, to change the tool.

FIG. 8 is a cross-sectional view through the canula of FIG. 7 but with the router inserted therein. The router 82 with its cutting edge 90 may be driven to rotate one way, and then another, i.e. to oscillate, e.g. to rotate precisely 360° clockwise, and then 360° counter-clockwise, and repeat. Typical cycle time for a rotation is 0.5 seconds or 120 oscillations per minute. Surgeons have long sought such a tool, as it is believed that it would improve cutting. As the router body 88 rotates one way and then the other, tissue that moves into the opening 90 is cut, and is then removed by a vacuum, and flushing of a saline solution through the aperture 92, which feeds ultimately to the hose 74.

It is understood that the oscillatory is not limited to routers, but may be used for drills, circular rasps, rotating scalpels, and a full range of motor driven and controlled tools.

FIG. 9 is a schematic side view of a surgical reciprocating rasp. The rasp 94 moves back and forth in a linear direction as shown by the arrows 96. It is connected at one end to a motor drive, which is a reciprocating motor or solenoid. The reciprocating motor would have a single Hall-effect sensor, which gives an indication of position. The control of the reciprocating rasp in the invention is done completely by the electrical system, and there are no springs connected to the rasp, and no mechanical resonance devices connected to the rasp. All of the force to move it to and fro is from the electrical control signal. The precise control for the reciprocating motion is achieved by having the control of the invention, provide a series of step control pulses, which force the linear solenoid motor output backward and forward. Each cycle may have a series of smaller pulses of uniform or different widths, as experimentation will indicate, to move the rasp firmly and accurately backward and forward. The tachometer feed back is then fed and a table look-up and the control can adjust for additional force to be applied, depending upon what is being cut or shaved by the tool. The wider the pulse, and the closer the pulses in each cycle are to each other, the more force that is applied. It is expected that to provide a smooth operation and to avoid possible vibration of the canula, the pulses close to the end and at the beginning of each cycle may be narrower than the pulses at the center of each cycle. In other words, the force of cutting can be controlled by the duty cycle, which would be adjustable throughout the surgical procedure, and as called for by measurement of the tachometer, and the output of the pulses. Again, it is emphasized that the control of the rasp is purely electrical without springs, without mechanical resonances, or other mechanical means.

A typical motion of a reciprocating rasp is about 0,6 cm, and with a cycle time of 1 second.

In another embodiment of the invention, the system provides two signals to the motor or solenoid. One, being a low frequency signal, e.g. with cycle time of one second, and the other being a high frequency signal of, e.g., with a cycle time of one millisecond. The low frequency signal is described as above, and the high frequency signal is substantially similar but more rapid. The compound signals give a compound motion to the reciprocating rasp, i.e. a dithering motion at high frequency with a short length, for example, in the range of 0,05 to 0,1 cm superimposed upon the slower stroke of approximately 0,6 cm. For certain surgical applications, this should prove advantageous. The control for both the high frequency and low frequency signal and the drive for them, would be a system as set forth herein.

It should be appreciated that the present invention is a control system for an electrical output, which drives for example, an electrical stepper or brushless motor with a rotating or reciprocating output. It provides precise control of both the force or torque, which the motor will produce, and also the velocity or speed at which the motor rotates or reciprocates. This is achieved due to the nature of the electrical output signal, and the table look-up in the controller, which table look-up can be adjusted easily and electronically, e.g. from a computer terminal for the various applications which the motor will be used, and the loads and degree of accuracy placed upon those motors.

It will be understood that each of the elements described above, or two or more together, also may find a useful application in other types of constructions differing from the types described above.

While the invention has been illustrated and described as embodied in an all-digital speed control system for brushless three-phase DC motor, it is not intended to be limited to the details shown, since various modifications and structural changes may be made without departing in any way from the scope of the present invention as defined in the following claims.

## Claims

1. An all-digital control system for a motor having an armature, comprising,
(a) a main digital signal processor for supplying a digital input command signal indicative of a desired motor operation;
(b) a drive controller in digital communication with the main processor, for generating, for each phase of the motor, and in response to the command signal, a digital commutation signal to move the armature, and a digital pulse width modulated signal having a duty cycle established by the input command signal;
(c) switching means in digital communication with the controller, for generating, for each phase, and in response to each commutation signal and each pulse width modulated signal, a digital two-state control signal having an on-state which lasts for said duty cycle;
(d) means in digital communication with the controller, for generating, for each phase, a digital tachometer signal indicative of armature position;
(e) said controller being further operative for processing the tachometer signal, to generate a digital output signal indicative of the actual armature speed or position, and for communicating the digital output signal to the main processor, wherein said controller includes a first look-up table having bit patterns corresponding to different armature positions and second look-up table for modulating said pulse width modulated signal.

2. The system according to claim 1, wherein the main processor and the drive controller are interconnected by, and digitally communicate through, a parallel bus or serial optical fibres.

3. The system according to claim 1, wherein the pulse width modulated signal has two states, and wherein the controller includes timer means having a timer output signal whose duration is established by the state of the pulse width modulated signal.

4. The system according to claim 1, wherein the controller includes watchdog timer means having a predetermined watchdog time, and wherein the controller includes shutdown means for ceasing generation of the commutation signals upon elapse of said watchdog time without receipt of the input command signal.

5. An all-digital speed control system for a motor having an armature, comprising:
(a) a main digital signal processor for supplying a digital input speed signal indicative of a desired armature speed;
(b) a drive controller in direct digital communication with the main processor, for generating, for each phase, and in response to the input speed signal, a digital commutation signal to move the armature, and a digital pulse width modulated signal having a duty cycle established by the input speed signal;
(c) switching means in digital communication with the controller, for generating, for each phase, and in response to each commutation signal and each pulse width modulated signal, a digital two-state speed control signal having an on-state which lasts for said duty cycle;
(d) means in direct digital communication with the controller, for generating, for each phase, a digital tachometer signal indicative of armature position;
(e) said controller being further operative for processing the tachometer signal, to generate a digital output speed signal indicative of the actual armature speed and for directly digitally communicating the output speed signal to the main processor, wherein said controller includes a first look up table having bit patterns corresponding to different armature positions and a second look up table for modulating said pulse width modulated signal.

6. The system according to claim 5, wherein the duty cycle of the pulse width modulated signal lies in an approximate range between 5% and 95%.

7. The system according to claim 5, wherein the pulse width modulated signal is carried on a signal carrier having a frequency of approximately 3.9 kHz.

8. The system according to claim 5, wherein the pulse width modulated signal has two states, and wherein the controller includes timer means having a timer output signal whose duration is established by the state of the pulse width modulated signal.

9. The system according to claim 5, wherein the controller includes watchdog timer means having a predetermined watchdog time, and wherein the controller includes shutdown means for ceasing generation of the commutation signals upon elapse of said watchdog time without receipt of the input speed signal.

10. The system according to claim 2, wherein a second-pulse width modulated signal is superimposed on said first pulse width modulated signal to provide a compound motion of said motor.

11. The system according to claim 10 further comprising a surgical instrument connection to said motor output whereby said compound motion is being conveyed to a surgical instrument.

12. The system according to claim 11 where said motor is a linear motor having fore and aft motion.

13. The system according to claim 12 wherein said instrument is a reciprocating rasp with said first signal driving said rasp with a stroke with about 0,6 cm, and said second signal devising said rasp with a stroke length of about 0,05 to 0,1 cm.

14. The system according to claim 12 wherein the force in each motion of said instrument is exclusively controlled by the duty cycle of each of said pulses.

15. The system according to claim 1, wherein the command signal is for oscillations of a rotary motor, and said motor further comprising an output for connection to a surgical instrument, and to which instrument the system inputs said oscillatory motion.

16. The system according to claim 15, wherein said surgical instrument is an oscillatory shaver.

17. The system according to claim 16, wherein said shaver oscillates 360° in each direction at a rate of approximately 12 rpm.

18. The system according to claim 1, wherein said motor is a rotary motor and comprises an output for connection to a surgical instrument, and said system further comprising an electric control for limiting torque applied to said output, with said torque limiter regulating the duty cycle of said pulse width modulating signal, whereby excessive torque is not applied to said instrument.

19. The system according to claim 18, wherein said torque limiter compares motor speed as generated from said digital output signal from said tachometer signal with the corresponding duty cycle of the control signal which comparison is proportional to torque.

## Patentansprüche

1. Volldigitales Steuersystem für einen Motor, der einen Anker besitzt, mit
(a) einem digitalen Haupt-Signalprozessor zum Bereitstellen eines digitalen Eingangsbefehlssignals, das den gewünschten Motorbetrieb anzeigt,
(b) einer Antriebssteuerung, die digital mit dem Hauptprozessor kommuniziert, um für jede Phase des Motors und auf das Befehlssignal ansprechend ein digitales Stromwendesignal zur Bewegung des Ankers und ein digitales, pulsbreitenmoduliertes Signal mit einer von dem Eingangsbefehlsignal festgelegten Tastrate zu erzeugen,
(c) einer Schalteinrichtung, die digital mit der Steuerung kommuniziert, um für jede Phase des Motors und auf jedes Stromwendesignal sowie jedes pulsbreitenmodulierte Signal ansprechend ein digitales, zweiwertiges Steuersignal zu erzeugen, das einen Einschaltzustand besitzt, der so lange wie die Tastrate dauert,
(d) Einrichtungen, die digital mit der Steuerung kommunizieren, um für jede Phase ein digitales Drehzahlmessersignal zu erzeugen, das die Ankerposition anzeigt,
(e) wobei die Steuerung weiterhin die Verarbeitung des Drehzahlmessersignals, um ein digitales Ausgangssignal, das die aktuelle Geschwindigkeit und Position des Ankers anzeigt, zu erzeugen, und die Kommunikation des digitalen Ausgangssignals an den Hauptprozessor durchführt, und die Steuerung eine erste Tabelle mit Bitmustern, die unterschiedlichen Positionen des Ankers wobeientsprechen, und eine zweite Tabelle zur Modulation des pulsbreitenmodulierten Signals enthält.

2. System nach Anspruch 1, dadurch gekennzeichnet, daß der Hauptprozessor und die Antriebssteuerung miteinander durch einen parallelen Bus oder serielle Lichtwellenleiter digital kommunizieren.

3. System nach Anspruch 1, dadurch gekennzeichnet, daß das pulsbreitenmodulierte Signal zwei Werte aufweist, und daß die Steuerung einen Zeitgeber mit einem Zeitgeberausgangssignal einschließt, dessen Dauer durch den Wert des pulsbreitenmodulierten Signals bestimmt wird.

4. System nach Anspruch 1, dadurch gekennzeichnet, daß die Steuerung einen Überwachungszeitgeber einschließt, der eine vorbestimmte Überwachungszeit aufweist, und daß die Steuerung eine Abschalteinrichtung für ein Unterlassen der Generierung von Stromwendesignalen, nachdem die Überwachungszeit ohne Empfang eines Eingangsbefehlssignals verstrichen ist, einschließt.

5. Volldigitales Geschwindigkeitssteuersystem für einen Motor, der einen Anker besitzt, mit
(a) einem digitalen Haupt-Signalprozessor zum Bereitstellen eines digitalen Geschwindigkeitseingangssignals, das die gewünschte Ankergeschwindigkeit anzeigt,
(b) einer Antriebssteuerung, die direkt digital mit dem Hauptprozessor kommuniziert, um für jede Phase und auf das Geschwindigkeitseingangssignal ansprechend ein digitales Stromwendesignal zur Bewegung des Ankers sowie ein digitales, pulsbreitenmoduliertes Signal mit einer von dem Geschwindigkeitseingangssignal festgelegten Tastrate zu erzeugen,
(c) einer Schalteinrichtung, die digital mit der Steuerung kommuniziert, um für jede Phase und auf jedes Stromwendesignal sowie jedes pulsbreitenmodulierte Signal ansprechend ein digitales, zweiwertiges Geschwindigkeitssteuersignal zu erzeugen, das einen Einschaltzustand besitzt, der solange wie die Tastrate dauert,
(d) Einrichtungen, die direkt digital mit der Steuerung kommunizieren, um für jede Phase ein digitales Drehzahlmessersignal zu erzeugen, das die Ankerposition anzeigt,
(e) wobei die Steuerung weiterhin die Verarbeitung des Drehzahlmessersignals, um ein digitales Geschwindigkeitsausgangssignal, das die aktuelle Ankergeschwindigkeit anzeigt, zu erzeugen, und die direkte digitale Kommunikation des digitalen Geschwindigkeitsausgangssignals mit dem Hauptprozessor durchführt, und die Steuerung eine erste Tabelle mit Bitmustern, die unterschiedlichen Positionen des Ankers entsprechen, und eine zweite Tabelle zur Modulation der pulsbreitenmodulierten Signale aufweist.

6. System nach Anspruch 5, dadurch gekennzeichnet, daß das die Tastrate des pulsbreitenmodulierten Signals im wesentlichen in einem Bereich zwischen 5% und 95% liegt.

7. System nach Anspruch 5, dadurch gekennzeichnet, daß das pulsbreitenmodulierte Signal durch ein Trägersignal mit einer Frequenz von ungefähr 3,9 kHz übertragen wird.

8. System nach Anspruch 5, dadurch gekennzeichnet, daß das pulsbreitenmodulierte Signal zwei Werte aufweist und die Steuerung einen Zeitgeber mit einem Zeitgeberausgangssignal aufweist, dessen Dauer durch den Wert des pulsbreitenmodulierten Signals festgelegt ist.

9. System nach Anspruch 5, dadurch gekennzeichnet, daß die Steuerung einen Überwachungszeitgeber einschließt, der eine bestimmte Überwachungszeit aufweist, und daß die Steuerung eine Abschalteinrichtung für ein Unterlassen der Generierung von Stromwendesignalen, nachdem die Überwachungszeit ohne Empfang eines Geschwindigkeitseingangssignals verstrichen ist, einschließt.

10. System nach Anspruch 2, dadurch gekennzeichnet, daß ein zweites pulsbreitenmoduliertes Signal dem ersten pulsbreitenmodulierten Signal überlagert ist, um eine zusammengesetzte Bewegung des Motors zu schaffen.

11. System nach Anspruch 10, dadurch gekennzeichnet, daß weiterhin eine Verbindung für ein chirurgisches Instrument mit dem Motorabtrieb vorgesehen ist, wobei die zusammengesetzte Bewegung auf ein chirurgisches Instrument übertragen wird.

12. System nach Anspruch 11, dadurch gekennzeichnet, daß der Motor ein Linearmotor mit einer Vor- und Zurückbewegung ist.

13. System nach Anspruch 12, dadurch gekennzeichnet, daß das Instrument als eine hin- und hergehende Raspel ausgebildet ist, wobei das erste Signal die Raspel mit einem Hub von ungefähr 0,6 cm antreibt und das zweite Signal die Raspel mit einer Hublänge von ungefähr 0,05 bis 0,1 cm versieht.

14. System nach Anspruch 12, dadurch gekennzeichnet, daß die Kraft bei jeder Bewegung des Instruments ausschließlich durch die Tastrate von jedem der Signale gesteuert ist.

15. System nach Anspruch 1, dadurch gekennzeichnet, daß das Befehlssignal für Oszillationen eines rotierenden Motors sorgt und daß der Motor weiterhin einen Abgang zur Verbindung mit einem chirurgischen Instrument aufweist, wobei an diesem Instrument die oszillatorische Bewegung des Systems anliegt.

16. System nach Anspruch 15, dadurch gekennzeichnet, daß das chirurgische Instrument als ein oszillierender Schaber ausgebildet ist.

17. System nach Anspruch 16, dadurch gekennzeichnet, daß der Schaber um 360° in jede Richtung bei einer Geschwindigkeit von ungefähr 12 U/min oszilliert.

18. System nach Anspruch 1, dadurch gekennzeichnet, daß der Motor als Rotationsmotor ausgebildet ist und einen Abgang zur Verbindung mit einem chirurgischen Instrument aufweist, und daß das System weiterhin eine elektrische Steuerung zur Begrenzung des Drehmoments an dem Abgang aufweist, wobei der Drehkraftbegrenzer die Tastrate des pulsbreitenmodulierten Signals reguliert, wodurch ein übermäßig hohes Drehmoment nicht an das Instrument angelegt wird.

19. System nach Anspruch 18, dadurch gekennzeichnet, daß der Drehkraftbegrenzer die Motorgeschwindigkeit wie sie aus dem digitalen Ausgangssignal von dem Drehzahlmessersignal erzeugt wird mit der entsprechenden Tastrate des Steuersignals vergleicht, wobei der Vergleich proportional zum Drehmoment ist.

## Revendications

1. Système de commande entièrement numérique pour un moteur possédant un induit, comprenant
(a) un processeur principal de signaux numériques pour délivrer un signal de commande d'entrée numérique indicatif d'un fonctionnement désiré du moteur;
(b) une unité de commande d'entraînement en communication numérique avec le processeur principal pour produire, pour chaque phase du moteur et en réponse au signal de commande, un signal de commutation numérique pour déplacer l'induit, et un signal numérique modulé selon une modulation d'impulsions en durée, possédant un cycle d'application fixé par le signal de commande d'entrée;
(c) des moyens de commutation en communication numérique avec l'unité de commande pour produire, pour chaque phase et en réponse à chaque signal de commutation et à chaque signal modulé selon une modulation d'impulsions en durée, un signal de commande numérique à deux états possédant un état appliqué qui dure pendant ledit cycle d'application;
(d) des moyens en communication numérique avec l'unité de commande pour produire pour chaque phase un signal tachymétrique numérique indicatif de la position de l'induit;
(e) ladite unité de commande agissant en outre de manière à traiter le signal tachymétrique pour produire un signal de sortie numérique indicatif de la vitesse ou de la position réelle de l'induit, et pour communiquer le signal de sortie numérique au processeur principal, ladite unité de commande comprenant une première table de consultation comportant des profils binaires correspondant à des positions différentes de l'induit, et une seconde table de consultation pour moduler ledit signal modulé selon une modulation d'impulsions en durée.

2. Système selon la revendication 1, dans lequel le processeur principal et l'unité de commande d'entraînement sont interconnectés et communiquent numériquement par l'intermédiaire d'un bus parallèle ou de fibres optiques en série.

3. Système selon la revendication 1, dans lequel le signal modulé selon une modulation d'impulsions en durée comporte deux états, et dans lequel l'unité de commande comprend des moyens formant minuterie, délivrant un signal de sortie de minuterie dont la durée est fixée par l'état du signal modulé selon une modulation d'impulsions en durée.

4. Système selon la revendication 1, dans lequel l'unité de commande comprend des moyens formant minuterie "chien de garde" contenant une durée de minutage prédéterminée, et dans lequel l'unité de commande comprend des moyens d'arrêt pour interrompre la production des signaux de commutation lors de l'écoulement de ladite durée de minutage sans réception du signal de commande d'entrée.

5. Système de commande de vitesse entièrement numérique pour un moteur comportant un induit, comprenant :
(a) un processeur principal de signaux numériques servant à délivrer un signal de vitesse d'entrée numérique indicatif d'une vitesse désirée de l'induit;
(b) une unité de commande d'entraînement en communication numérique directe avec le processeur principal pour produire, pour chaque phase et en réponse au signal de vitesse d'entrée, un signal de commutation numérique pour déplacer l'induit, et un signal numérique modulé selon une modulation d'impulsions en durée et possédant un cycle d'application fixé par le signal de vitesse d'entrée;
(c) des moyens de commutation en communication numérique avec l'unité de commande pour produire, pour chaque phase et en réponse à chaque signal de commutation et à chaque signal modulé selon une modulation d'impulsions en durée, un signal numérique de commande de vitesse à deux états possédant un état appliqué qui dure pendant ledit cycle d'application;
(d) des moyens en communication numérique directe avec l'unité de commande pour produire, pour chaque phase, un signal de tachymètre numérique indicatif de la position de l'induit;
(e) ladite unité de commande agissant en outre de manière à traiter le signal tachymétrique pour produire un signal de vitesse de sortie numérique indicatif de la vitesse réelle de l'induit, et servant à transmettre directement, de façon numérique, le signal de vitesse de sortie au processeur principal, ladite unité de commande comprenant une première table de consultation possédant des profils binaires correspondant à des positions différentes de l'induit, et une seconde table de consultation servant à moduler ledit signal modulé selon une modulation d'impulsions en durée.

6. Système selon la revendication 5, dans lequel le cycle d'application du signal modulé selon une modulation d'impulsions en durée se situe dans une gamme approximative comprise entre 5 % et 95 %.

7. Système selon la revendication 5, dans lequel le signal modulé selon une modulation d'impulsions en durée est porté par une porteuse du signal qui possède une fréquence d'environ 3,9 kHz.

8. Système selon la revendication 5, dans lequel le signal modulé selon une modulation d'impulsions en durée comporte deux états, et dans lequel l'unité de commande comprend des moyens formant minuterie possédant un signal de sortie de minuterie, dont la durée est fixée par l'état du signal modulé selon une modulation d'impulsions en durée.

9. Système selon la revendication 5, dans lequel l'unité de commande comprend des moyens formant minuterie "chien de garde" possédant une durée de minutage prédéterminée et dans lequel l'unité de commande comprend des moyens d'interruption pour arrêter la production des signaux de commutation lors de l'écoulement de la durée de minutage sans réception du signal de vitesse d'entrée.

10. Système selon la revendication 2, dans lequel un second signal modulé selon une modulation d'impulsions en durée est superposé audit premier signal modulé selon une modulation d'impulsions en durée pour produire un mouvement composite dudit moteur.

11. Système selon la revendication 10, comprenant en outre la connexion d'un instrument chirurgical à ladite sortie du moteur, ce qui a pour effet que ledit déplacement composite est appliqué à l'instrument chirurgical.

12. Système selon la revendication 11, dans lequel ledit moteur est un moteur linéaire possédant un déplacement alternatif d'avant en arrière.

13. Système selon la revendication 12, selon lequel ledit instrument est une râpe se déplaçant en va-et-vient, ledit premier signal commandant ladite râpe selon une course d'environ 0,6 cm et ledit second signal entraînant ladite râpe sur une course d'une longueur d'environ 0,05 à 0,1 cm.

14. Système selon la revendication 12, dans lequel la force lors de chaque déplacement dudit instrument est commandée exclusivement par le cycle d'application de chacune desdites impulsions.

15. Système selon la revendication 1, dans lequel le signal de commande sert à commander des oscillations d'un moteur rotatif, et ledit moteur comporte en outre une sortie destinée à être connectée à un instrument chirurgical, le système appliquant ledit déplacement oscillatoire à cet instrument.

16. Système selon la revendication 15, dans lequel ledit instrument chirurgical est un rasoir oscillant.

17. Système selon la revendication 16, dans lequel ledit rasoir oscille sur 360° dans chaque direction à une vitesse d'environ 12 tr/mn.

18. Système selon la revendication 1, dans lequel ledit moteur est un moteur rotatif et comporte une sortie pour sa connexion à un instrument chirurgical, et ledit système comporte en outre une commande électrique pour limiter le couple appliqué à ladite sortie, ledit limiteur de couple réglant le cycle d'application dudit signal de modulation d'impulsions en durée, ce qui a pour effet qu'aucun couple excessif n'est appliqué audit instrument.

19. Système selon la revendication 18, dans lequel ledit limiteur de couple compare la vitesse du moteur telle qu'elle est produite par ledit signal de sortie à partir dudit signal tachymétrique avec le cycle correspondant d'application du signal de commande, laquelle comparaison est proportionnelle au couple.
